# EUROPEAN PATENT SPECIFICATION

(11) **EP 1 560 568 B1**
(45) Date of publication and mention of the grant of the patent: **26.03.2014**
(21) Application number: 03755718.8
(22) Date of filing: 06.10.2003
(51) Int. Cl.: A61K 9/16, A61K 9/22, A61K 31/7048

(54) **CONTROLLED RELEASE PHARMACEUTICAL COMPOSITIONS CONTAINING SODIUM ALGINATE AND SODIUM CALCIUM ALGINATE**
PHARMAZEUTISCHE ZUSAMMENSETZUNGEN MIT VERZÖGERTER WIRKSTOFFABGABE ENTHALTEND NATRIUMALGINAT UND NATRIUMCALCIUMALGINAT
COMPOSITIONS PHARMACEUTIQUES A LIBERATION CONTROLEE CONTENANT DE L'ALGINATE DE SODIUM ET DE L'ALGINATE DE SODIUM ET CALCIUM

(30) Priority: 08.10.2002 SI 200200242
(43) Date of publication of application: 10.08.2005
(73) Proprietor: KRKA, tovarna zdravil, d.d., Novo mesto, 8501 Novo mesto (SI)
(72) Inventor: PISEK, Robert, SI-1000 Ljubljana (SI); KRAMAR, Andrejka, SI- 8000 Novo mesto (SI); VRECER, Franc, SI- 8351 Straza (SI); KINCL, Maja, SI- 8000 Novo mesto (SI); BREZNIK, Marjanca, SI- 1000 Ljubljana (SI)
(74) Representative: UEXKÜLL & STOLBERG
(86) International application number: PCT/SI2003/000034
(87) International publication number: WO 2004/032904

(56) References cited:
- EP-A- 0 188 040
- EP-A- 0 275 336
- WO-A-97/22335
- WO-A-98/56357
- NICHOLSON S.J. ET AL.: 'Investigation of drug release from sodium alginate-sodium calcium alginate matrices' J. PHARMACY AND PHARMACOLOGY vol. 42, 1990, page 2P, XP009028004

## Description

### FIELD OF THE INVENTION

The present invention relates to pharmaceutical compositions, which contain alginates and are able to release an active ingredient in a controlled, but sufficiently fast manner. In particular, the compositions are such that they offer a once-daily dose regimen of a poorly soluble drug to a patient in need thereof.

The invention also relates to a process for the preparation of such compositions.

### BACKGROUND OF THE INVENTION

Controlled release formulations allow the possibility of reducing the dosage regimens for drugs, especially those administered orally to patients. The advantages of reduced dosage regimens for the patients are convenience, a better assurance of compliance and a reduction of severity and frequency of side effects as they maintain substantially constant blood levels and avoid the fluctuations associated with the conventional immediate release formulations administered more than once a day.

Controlled release can generally be achieved by using specific materials as matrix forming agents or coating agents in a dosage form. In particular, the use of alginates as matrix forming agents for such retardation of the drug release has already been disclosed in the prior art.

EP-A-0 188 040 (Abbott) describes controlled release solid dosage forms having a sodium and sodium-calcium alginate matrix. The sodium alginate is usually present in amounts that are higher than those of the less soluble sodium-calcium alginate. Moreover, it is described that the *in vitro* dissolution rate decreases when increasing the quantity of sodium-calcium alginate while keeping the amount of sodium alginate constant.

WO 97/22335 (Abbott) discloses controlled release matrix formulations of poorly soluble basic drugs. The formulations comprise a water-soluble alginate salt, a complex salt of alginic acid and an effective amount of an organic carboxylic acid. Exemplified compositions comprise sodium alginate and sodium-calcium alginate in a weight ratio of 8:1. The organic carboxylic acid serves to facilitate dissolution of the drugs at a higher pH. Formulations without an organic acid result in a slow release of poorly soluble basic drugs.

WO 02/17885 (Ranbaxy) discloses once-daily controlled release formulations of erythromycin derivatives. The formulation comprises rate-controlling polymers such as carbohydrate gums, polyuronic acid salts like sodium alginate, cellulose ethers or acrylic acid polymers in an amount of 0.1 to 4% as a matrix former. The formulation may further comprise gas generating components alone or in combination with an organic acid, swelling agents, lubricants and fillers. The use of small amounts of rate controlling polymers ensures that the total weight of the formulations is low.

However, conventional formulations described in EP-A-0 188 040 suffer from the drawbacks of slow release from an alginate matrix and of poor bioavailability, particularly of poorly water-soluble basic drugs such as erythromycin derivatives. Erythromycin derivatives, especially clarithromycin, show a low solubility in the lower intestine where a pH of 6 to 8 prevails.

In the formulations of the other two publications carboxylic acids were used as substances that improve the solubility of clarithromycin in the lower intestine. However, clarithromycin is sensitive to acid environment. Formulations without acids were developed in order to overcome the stability problem of erythromycin derivative in dosage forms.

### DETAILED DESCRIPTION OF THE INVENTION

According to the present invention these problems are solved by a controlled release pharmaceutical composition, which comprises
(a) at least one active ingredient,
(b) sodium alginate, and
(c) sodium-calcium alginate,
and wherein the weight ratio of sodium alginate (b) to sodium-calcium alginate (c) is from 1:1.1 to 1:2.

We have surprisingly found that the drawbacks of the conventional formulations can be eliminated by using the above ratio of sodium alginate to sodium-calcium alginate, which provides for a higher amount of sodium-calcium alginate compared to sodium alginate. It is very surprising that especially the problem of slow release of the active ingredient is solved by employing higher amounts of sodium-calcium alginate since this alginate is, according to the prior art, known to be responsible for retarding the drug release from the dosage form and is therefore generally used in smaller amounts compared to the more soluble sodium alginate.

The active ingredient (a) of the composition according to the invention is generally a basic drug showing a low solubility in water of less than 1 part by weight in 30 parts by weight of water. It is preferred that the active ingredient is an erythromycin derivative and in particular clarithromycin.

The composition usually comprises 1 to 75 % by weight and preferably 30 to 60 % and even more preferably 45 to 55 % by weight of the active ingredient.

The composition according to the invention also includes sodium alginate (b) and sodium-calcium alginate (c) in the above-mentioned weight ratio.

It is preferred that the composition comprises 5 to 15 % by weight of sodium alginate and/or comprises 5.5 to 20 % by weight of sodium-calcium alginate.

Especially preferred compositions according to the invention contain about 7 to 10 % by weight of sodium alginate and 8 to 12 % by weight of sodium-calcium alginate.

It has also been found that particularly preferred compositions further comprise (d) at least one surfactant. Such compositions have shown a very desirable release behaviour.

The surfactant may be selected among non-ionic or ionic surfactants or mixtures thereof.

Suitable non-ionic surfactants are selected from the group of alkylglucosides, alkylmaltosides, alkylthioglucosides, lauryl macrogolglycerides, polyoxyethylene alkylphenols, polyoxyethylene alkylethers, polyethylene glycol fatty acid esters, polyethylene glycol glycerol fatty acid esters, polyoxyethylene sorbitan fatty acid esters, polyoxyethylene-polyoxypropylene block copolymers, polyglyceryl fatty acid esters, polyoxyethylene glycerides, polyoxyethylene vegetable oils, polyoxyethylene hydrogenated vegetable oils, sterols, and mixtures thereof. The preferred non-ionic surfactants are polyoxyethylene sorbitan fatty acid esters, which are sold under the trade names Polysorbate or Tween. Specific examples are Polysorbate 80 or Polysorbate 20. Suitable ionic surfactants are selected from the group of fatty acid salts, bile salts, phospholipides, phosphoric acid esters, carboxylates, sulphates, sulphonates and mixture thereof. A specific example is sodium lauryl sulphate.

The phamiaceutical composition according to the invention usually comprises 0.5 to 10 % and preferably 1 to 3 % by weight of a surfactant.

The controlled release composition of the invention may also include further pharmaceutically acceptable excipients. Suitable excipients are diluents, e.g. lactose or microcrystalline cellulose; binders, e.g. polyvinyl pyrrolidone, hydroxypropyl cellulose or hydroxypropylmethyl cellulose; lubricants, e.g. talc, stearic acid or magnesium stearate; glidants e.g. silica, colloidal anhydrous; colouring agents; and pH modifiers.

The composition according to the invention is preferably produced using conventional dry or wet granulation procedure and is in the form of a solid oral dosage form with tablets being preferred. It is also possible that the composition takes the form of granules or pellets, which may be filled in a capsule.

Any suitable oral dosage form may additionally be coated with usual coating substances such as polymers. Such coating may provide for an additional or modifying controlled release effect or may serve to mask the taste or odour or to improve the stability of the dosage form.

Finally, the invention also relates to a process for preparing the composition, which comprises the providing of a mixture of sodium alginate (a) and sodium-calcium alginate (b) and combining this mixture with the active ingredient (a) and optionally with a surfactant (d).

The pharmaceutical composition according to the invention is particularly suitable for a once-daily administration of erythromycin derivatives such as clarithromycin, as it shows the necessary release for such purpose and is superior to conventional formulations based on alginates due to a faster release rate without the need of carboxylic acids.

The invention is further illustrated in the continuation by means of examples.

### EXAMPLES

### Examples 1 to 8

### TABLET MANUFACTURING PROCEDURE

Tablets were prepared using the components as given in Table 1 below. In the Table 1 there are given compositions of different formulations of clarithromycin controlled release tablets prepared in the Examples 1 - 8 by the following procedure.

The drug clarithromycin and the other excipients except the surfactant Polysorbate 80 were screened. The screened materials except lubricants were dry- blended and granulated with water or an aqueous solution of the surfactant. The obtained wet granulate was dried until the granulate had a moisture content of less than 3 % (w/w) by weight of water. The dried granules were screened and mixed with the lubricants and glidants, i.e. stearic acid, magnesium stearate, talc and silica, colloidal anhydrous. The resulting mixture was then compressed using a rotary tabletting machine to give tablets, which were subsequently coated with a hydroxypropylmethyl cellulose dispersion to cover the unpleasant taste of clarithromycin.

**Table 1: Compositions of different formulations of clarithromycin controlled release tablets**

| Example | 1 | 2 | 3 | 4 | 5 | 6 | 7 | 8 |
|---|---|---|---|---|---|---|---|---|
| Ingredient | mg/ tab | mg/ tab | mg/ tab | mg/ tab | mg/ tab | mg/ tab | mg/ tab | mg/ tab |
| Clarithromycin | 500 | 500 | 500 | 500 | 500 | 500 | 500 | 500 |
| Sodium alginate | 80 | 80 | 80 | 80 | 80 | 80 | 80 | 80 |
| Sodium-calcium alginate | 30 | 30 | 30 | 90 | 90 | 120 | 60 | 90 |
| Lactose | 289 | 269 | 259 | 229 | 199 | 169 | 229 | 225 |
| Polyvinylpyrrolidone | 30 | 30 | 30 | 30 | 30 | 30 | 30 | 30 |
| Polysorbate 80 | - | 20 | 30 | - | 30 | 30 | 30 | 30 |
| Stearic acid | 21 | 21 | 21 | 21 | 21 | 21 | 21 | - |
| Magnesium stearate | 20 | 20 | 20 | 20 | 20 | 20 | 20 | 10 |
| Silica, colloidal anhydrous | - | - | - | - | - | - | - | 5 |
| Talc | 30 | 30 | 30 | 30 | 30 | 30 | 30 | 30 |

### DISSOLUTION TESTS

The tablets according to examples 1 and 4 and Klacid UNO (Ch. Nr.: 81198TF02) (Abbott) were subjected to a dissolution test. The dissolution test was carried out in 900 ml of acetate buffer (USP) having a pH of 5.0 using USP apparatus II at 50 rpm.

| Formulation / Time (h) | 1 | 4 | Klacid UNO (Ch. Nr.: 81198TF02) |
|---|---|---|---|
| 0 | 0 | to | 0 |
| 2 | 7 | 11 | 8 |
| 4 | 16 | 22 | 20 |
| 6 | 25 | 35 | 32 |
| 8 | 32 | 48 | 43 |
| 12 | 44 | 67 | 60 |
| 24 | 69 | 93 | 88 |

In Example 1, where the formulation was such as suggested and claimed in WO 97/22335 but without citric acid, the dissolved amount of clarithromycin after 12 hours was less than 50 %. However, in Example 4 more than 50 % of clarithromycin were dissolved after 12 hours and the release profile was very similar to the release profile of Klacid UNO, which comprises citric acid.

## Claims

1. A controlled release pharmaceutical composition, which comprises
(a) at least one active ingredient,
(b) sodium alginate, and
(c) sodium-calcium alginate,
wherein the weight ratio of sodium alginate (b) to sodium-calcium alginate (c) is from 1:1.1 to 1:2.

2. Pharmaceutical composition according to claim 1, wherein the active ingredient (a) is a basic drug showing a low solubility in water of less than 1 part by weight in 30 parts by weight of water.

3. Pharmaceutical composition according to claim 1 or 2, wherein the active ingredient (a) is an erythromycin derivative.

4. Pharmaceutical composition according to claim 3, wherein the erythromycin derivative is clarithromycin.

5. Pharmaceutical composition according to any one of claims 1 to 4, which comprises
(a) 1 to 75 % by weight and preferably 30 to 60 % and even more preferably 45 to 55 % by weight of the active ingredient.

6. Pharmaceutical composition according to any one of claims 1 to 5, which comprises
(b) 5 to 15 % by weight of sodium alginate, preferably 7 to 10 % by weight of sodium alginate.

7. Pharmaceutical composition according to any one of claims 1 to 6, which comprises
(c) 5.5 to 20 % by weight of sodium-calcium alginate, preferably 8 to 12 % by weight of sodium-calcium alginate.

8. Pharmaceutical composition according to any one of claims 1 to 7, which comprises
(d) at least one surfactant.

9. Pharmaceutical composition according to claim 8, wherein the surfactant is polyoxyethylene sorbitan fatty acid ester and preferably Polysorbate 80.

10. Pharmaceutical composition according to claim 8 or 9, which comprises
(d) 0.5 to 10 % and preferably 1 to 3 % by weight of the surfactant.

11. Process for preparing the composition according to any one of claims 1 to 10, which comprises the providing of a mixture of sodium alginate (b) and sodium-calcium alginate (c) and combining this mixture with the active ingredient (a) and optionally with the surfactant (d).

## Patentansprüche

1. Pharmazeutische Zusammensetzung mit gesteuerter Freisetzung, die
(a) mindestens einen Wirkstoff,
(b) Natriumalginat und
(c) Natrium-Calciumalginat enthält,
wobei das Gewichtsverhältnis von Natriumalginat (b) zu Natrium-Calciumalginat (c) von 1:1.1 bis 1:2 ist.

2. Pharmazeutische Zusammensetzung nach Anspruch 1, bei der der Wirkstoff (a) ein basischer Wirkstoff ist, der eine niedrige Löslichkeit in Wasser von weniger als 1 Gewichtsteil in 30 Gewichtsteilen Wasser aufweist.

3. Pharmazeutische Zusammensetzung nach Anspruch 1 oder 2, bei der der Wirkstoff (a) ein Erythromycinderivat ist.

4. Pharmazeutische Zusammensetzung nach Anspruch 3, bei der das Erythromycinderivat Clarithromycin ist.

5. Pharmazeutische Zusammensetzung nach einem der Ansprüche 1 bis 4, die
(a) 1 bis 75 Gew.-% und bevorzugt 30 bis 60 Gew.-% und ganz besonders bevorzugt 45 bis 55 Gew.-% des Wirkstoffes enthält.

6. Pharmazeutische Zusammensetzung nach einem der Ansprüche 1 bis 5, die
(b) 5 bis 15 Gew.-% Natriumalginat, vorzugsweise 7 bis 10 Gew.-% Natriumalginat
enthält.

7. Pharmazeutische Zusammensetzung nach einem der Ansprüche 1 bis 6, die
(c) 5,5 bis 20 % Natrium-Calciumalginat, vorzugsweise 8 bis 12 Gew.-% Natrium-Calciumalginat
enthält.

8. Pharmazeutische Zusammensetzung nach einem der Ansprüche 1 bis 7, die
(d) mindestens ein Tensid
enthält.

9. Pharmazeutische Zusammensetzung nach Anspruch 8, bei der das Tensid Polyoxyethylensorbitanfettsäureester und vorzugsweise Polysorbate 80 ist.

10. Pharmazeutische Zusammensetzung nach Anspruch 8 oder 9, die
(c) 0,5 bis 10 % und vorzugsweise 1 bis 3 Gew.-% des Tensids
enhält.

11. Verfahren zur Herstellung der Zusammensetzung nach einem der Ansprüche 1 bis 10, bei dem eine Mischung von Natriumalginat (b) und Natrium-Calciumalginat (c) zur Verfügung gestellt wird und diese Mischung mit dem Wirkstoff (a) und ggf. mit dem Tensid (d) kombiniert wird.

## Revendications

1. Composition pharmaceutique à libération régulée, qui comporte :
a) au moins un ingrédient actif,
b) de l'alginate de sodium,
c) et de l'alginate de sodium et de calcium,
et dans laquelle le rapport pondéral de l'alginate de sodium (b) à l'alginate de sodium et de calcium (c) vaut de 1/1,1 à 1/2.

2. Composition pharmaceutique conforme à la revendication 1, dans laquelle l'ingrédient actif (a) est un médicament basique qui présente une solubilité dans l'eau faible, valant moins de 1 partie en poids dans 30 parties en poids d'eau.

3. Composition pharmaceutique conforme à la revendication 1 ou 2, dans laquelle l'ingrédient actif (a) est un dérivé d'érythromycine.

4. Composition pharmaceutique conforme à la revendication 3, dans laquelle le dérivé d'érythromycine est de la clarithromycine.

5. Composition pharmaceutique conforme à l'une des revendications 1 à 4, qui comprend
a) de 1 à 75 % en poids, de préférence de 30 à 60 % et mieux encore de 45 à 55 % en poids de l'ingrédient actif.

6. Composition pharmaceutique conforme à l'une des revendications 1 à 5, qui comprend
b) de 5 à 15 % en poids d'alginate de sodium, et de préférence, de 7 à 10 % en poids d'alginate de sodium.

7. Composition pharmaceutique conforme à l'une des revendications 1 à 6, qui comprend
c) de 5,5 à 20 % en poids d'alginate de sodium et de calcium, et de préférence, de 8 à 12 % en poids d'alginate de sodium et de calcium.

8. Composition pharmaceutique conforme à l'une des revendications 1 à 7, qui comprend
d) au moins un agent tensioactif.

9. Composition pharmaceutique conforme à la revendication 8, dans laquelle l'agent tensioactif est un ester d'acide gras et de sorbitanne polyéthoxylé, et de préférence du Polysorbate 80.

10. Composition pharmaceutique conforme à la revendication 8 ou 9, qui comprend
d) de 0,5 à 10 % et de préférence de 1 à 3 % en poids d'agent tensioactif.

11. Procédé de préparation d'une composition conforme à l'une des revendications 1 à 10, qui comprend le fait de prendre un mélange d'alginate de sodium (b) et d'alginate de sodium et de calcium (c), et le fait de combiner ce mélange avec l'ingrédient actif (a), et en option, avec l'agent tensioactif (d).
